# EUROPEAN PATENT SPECIFICATION

(11) **EP 3 219 270 B1**
(45) Date of publication and mention of the grant of the patent: **05.05.2021**
(21) Application number: 17160306.1
(22) Date of filing: 10.03.2017
(51) Int. Cl.: A61B 17/70

(54) **TRANSVERSE CONNECTORS FOR SPINAL SYSTEMS**
QUERVERBINDER FÜR WIRBELSÄULENSYSTEME
CONNECTEURS TRANSVERSAUX POUR SYSTÈMES SPINAUX

(30) Priority: 16.03.2016 US 201615071437
(43) Date of publication of application: 20.09.2017
(73) Proprietor: Globus Medical, Inc., Audubon, PA 19403 (US)
(72) Inventor: WOLFE, Daniel, Hatfield, PA-19440 (US); SPANGLER, Daniel, Green Lane, PA-18054 (US); CIANFRANI, Jason, East Norriton, PA-19401 (US)
(74) Representative: Morabito, Sara

(56) References cited:
- EP-A1- 0 778 007
- EP-A1- 1 762 195
- EP-A1- 2 962 652
- US-A- 5 487 742

## Description

### FIELD OF THE INVENTION

The present disclosure is generally directed to transverse connectors for use in stabilizing the spine.

### BACKGROUND

Many types of spinal irregularities can cause pain, limit range of motion, or injure the nervous system within the spinal column. These irregularities can result from, without limitation, trauma, tumor, disc degeneration, and disease. Often, these irregularities are treated by immobilizing a portion of the spine. This treatment typically involves affixing a plurality of screws and/or hooks to one or more vertebrae and connecting the screws or hooks to an elongate rod that generally extends in the direction of the axis of the spine.

Treatment for these spinal irregularities often involves using a system of pedicle screws and rods to attain stability between spinal segments. Instability in the spine can create stress and strain on neurological elements, such as the spinal cord and nerve roots. In order to correct this, implants of certain stiffness can be implanted to restore the correct alignment and portion of the vertebral bodies. In many cases, an anchoring member such as a pedicle screw along with a vertical solid member can help restore spinal elements to a pain free situation, or at least may help reduce pain or prevent further injury to the spine, such as described in EP 0 778 007 and EP 2 962 652.

There is a need for a transverse connector (a.k.a. transconnector) that connects two rod systems that are positioned on opposing sides of the spine. There is also a need for a transverse connector that provides stability to the spinal implant construct as well as being smaller in profile so as not to interfere with adjacent screw or the spinal cord.

### SUMMARY

The present application describes various systems and devices related to transverse connectors. In some embodiments, a surgical system comprises a first rod member, a second rod member, and a transverse connector operably attached to the first rod member and the second rod member. The transverse connector comprises a first sub-assembly for gripping onto the first rod member and a second sub-assembly for gripping onto the second rod member, wherein the first rod member is bottom loaded onto the first sub-assembly and the second rod member is bottom loaded onto the second sub-assembly.

In some embodiments, a surgical system comprises a first rod member, a second rod member, and a transverse connector operably attached to the first rod member and the second rod member. The transverse connector comprises a first sub-assembly for gripping onto the first rod member, a second sub-assembly for gripping onto the second rod member and a cross rod, wherein at least one of the first sub-assembly and the second sub-assembly is slidable along the cross rod.

According to a further aspect of the invention it is provided a surgical system comprising: a first rod member; a second rod member; and a transverse connector operably attached to the first rod member and the second rod member, wherein the transverse connector comprises a first sub-assembly for gripping onto the first rod member, a second sub-assembly for gripping onto the second rod member and a cross rod, wherein at least one of the first sub-assembly and the second sub-assembly is slidable along the cross rod.

In a version the cross rod comprises a first slot for receiving the first sub-assembly and a second slot for receiving the second sub-assembly.

In a further version the first sub-assembly comprises an inner clamp and an outer clamp.

In another version the outer clamp of the first sub-assembly comprises a rail that is slidable within a slot formed in the cross rod.

In an embodiment the first sub-assembly further comprises a nut, wherein the nut extends around an upper portion of the inner clamp.

In a further embodiment the inner clamp comprises an upper threaded portion and a lower portion comprising a pair of fingers.

In another version the cross rod comprises an arched portion.

In another version the at least one of the first sub-assembly and the second sub-assembly is slidable along a slot formed in the cross rod.

The at least one of the first sub-assembly and the second sub-assembly may be slidable up to 3 mm.

The at least one of the first sub-assembly and the second sub-assembly may comprise a rail that extends through the slot formed in the cross rod.

### BRIEF DESCRIPTION OF THE DRAWINGS

These drawings illustrate certain aspects of the present invention and should not be used to limit or define the invention.
FIG. 1 shows an embodiment of a stabilization system using a transverse connector in accordance with some embodiments.
FIG. 2 shows a perspective view of a transverse connector in accordance with some embodiments.
FIG. 3 shows a side view of the transverse connector of FIG. 2.
FIG. 4 shows a side cross-sectional view of the transverse connector of FIG. 2.
FIG. 5 shows a top view of the transverse connector of FIG. 2.
FIG. 6 shows a front cross-sectional view of the transverse connector of FIG. 2.
FIG. 7 shows a side cross-sectional view of the transverse connector of FIG. 2, whereby the sub-assemblies including the inner clamps are separated a first distance.
FIG. 8 shows a side cross-sectional view of the transverse connector of FIG. 2, whereby the sub-assemblies including the inner clamps are separated a second distance different from the first distance in FIG. 7.
FIGS. 9A-9E show different views of individual components of the transverse connector of FIG. 2.
FIG. 10 shows an embodiment of a stabilization system using an alternative transverse connector in accordance with some embodiments.
FIG. 11 shows a perspective view of an alternative transverse connector in accordance with some embodiments.
FIG. 12 shows a side view of the transverse connector of FIG. 11.
FIG. 13 shows a top view of the transverse connector of FIG. 11.
FIG. 14 shows a side cross-sectional view of a clamp body of the transverse connector of FIG. 11.
FIG. 15 shows a front cross-sectional view of a clamp body of the transverse connector of FIG. 11.
FIG. 16 shows top cross-sectional view of a clamp body of the transverse connector of FIG. 11.
FIG. 17 shows a side cross-sectional view of the transverse connector of FIG. 11 without rod members received therein.
FIG. 18 shows a side cross-sectional view of the transverse connector of FIG. 11 with rod members received therein.
FIG. 19 shows an alternative cross-connector in accordance with some embodiments.

### DETAILED DESCRIPTION OF THE ILLUSTRATED EMBODIMENTS

The present application is directed to systems and devices related to transverse connectors used to connect two rod members, according to the invention as defined in independent claim 1. Preferred embodiments of the invention are defined in the dependent claims.

In spinal stabilization systems utilizing rod members connected by a transverse connector, it is often difficult to determine an appropriate transverse connector to use, as the distance of separation can vary between the rod members along the length of the spine. Furthermore, in some situations, the rod members can be non-parallel to one another, further making it difficult to find an appropriate transverse connector that can accommodate each of the rod members. Advantageously, the transverse connector systems described herein are capable of accommodating rod members having varying distances between them, as well as rod members that may be at a non-parallel angle relative to one another.

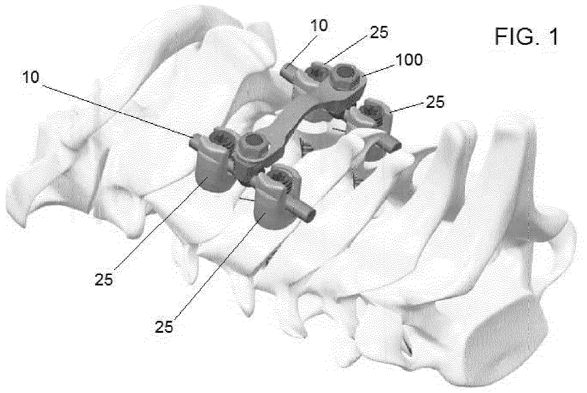

FIG. 1 shows an embodiment of a stabilization system using a transverse connector in accordance with some embodiments. The stabilization system comprises a first rod member 10 received in first and second screws (e.g., pedicle screws) 25, and a second rod member 10 received in third and fourth screws (e.g., pedicle screws) 25. The first rod member 10 is positioned on one side of a spine, while the second rod member 10 is positioned on the other side of the spine. An improved transverse connector 100 extends between the first rod member 10 and the second rod member 10. In some embodiments, the transverse connector 100 can be delivered on top of the first rod member 10 and the second rod member 10. The transverse connector 100 is advantageously capable of accommodating rod members having varying lengths of separation, as well as rod members that are non-parallel to one another.

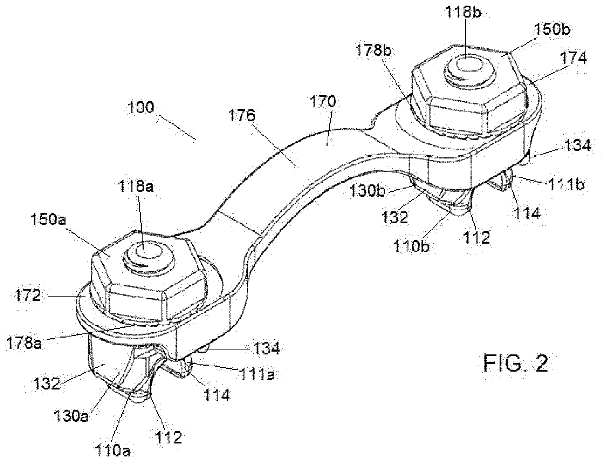

FIG. 2 shows a perspective view of a transverse connector in accordance with some embodiments. On one end, the transverse connector 100 comprises a first sub-assembly comprising a first inner clamp 110a for receiving a first rod member, a first outer clamp 130a, and a first nut 150a. On the other end, the transverse connector 100 comprises a second sub-assembly comprising a second inner clamp 110b for receiving a second rod member, a second outer clamp 130b, and a second nut 150b. The first sub-assembly and the second sub-assembly are connected to one another via a cross rod 170 that extends therebetween.

The first sub-assembly comprises a first inner clamp 110a, a first outer clamp 130a and a first nut 150a. The first inner clamp 110a is comprised of a lower portion 111a and an upper portion 118a. The lower portion 111a comprises a pair of tongs or fingers 112, 114 that are capable of gripping a first rod member 10 therebetween. In some embodiments, the inner clamp 110a is capable of being top-loaded onto a first rod member. The first rod member can also be viewed as being bottom-loaded into the inner clamp 110a. The upper portion 118a of the inner clamp 110a comprises a threaded cylindrical portion that is capable of engaging inner threads of the first nut 150a. Rotation of the first nut 150a causes the fingers 112, 114 of the inner clamp 110a to close on the first rod member, as will be discussed in further detail below. In some embodiments, an upper section of the threaded upper portion 118a is distorted or peened over, thereby preventing a nut 150a from loosening from the threaded upper portion 118a.

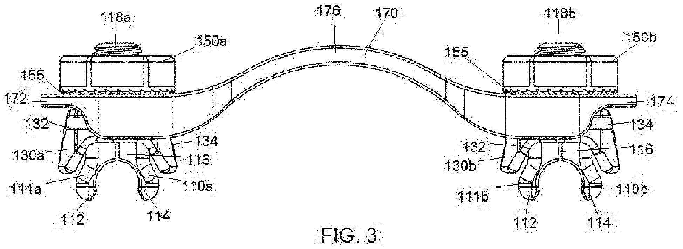

The first outer clamp 130a comprises a first clamping portion 132 and a second clamping portion 134. As shown in FIG. 3, the first clamping portion 132 comprises an inner wall designed to contact an outer surface of the finger 112 of the first inner clamp 110a, while the second clamping portion 134 comprises an inner wall designed to contact an outer surface of the finger 114 of the first inner clamp 110a. Rotation of the first nut 150a causes the first inner clamp 110a to be drawn upward. As the first inner clamp 110a is drawn upward, the inner walls of the first outer clamp 130a engage the outer walls of the first inner clamp 110a, thereby compressing the first inner clamp 110a onto a first rod member that is received therein.

The first nut 150a comprises an inner threaded section that is configured to engage the outer threads of the upper portion 118a of the first inner clamp 110a. In some embodiments, rotation of the first nut 150a in a first direction draws the first inner clamp 110a upward toward the inner walls of the first outer clamp 130a, thereby causing the fingers 112, 114 of the first inner clamp 110a to be compressed onto a first rod member 10. Rotation of the first nut 150a in a second opposite direction translates the first inner clamp 110a downward and away from the inner walls of the first outer clamp 130a, thereby causing the fingers 112, 114 of the first inner clamp 110a to release from the first rod member 10 if desired. In some embodiments, an upper surface of the first nut 150a is substantially smooth, while a lower surface of the first nut 150a comprises a more textured surface. In some embodiments, the lower surface of the first nut 150a comprises a star grind. Advantageously, when the first nut 150a is fully tightened, the star grind provides resistance against an upper surface of the cross rod 170, thereby reducing the likelihood of the first nut 150a unintentionally rotating backwards and loosening on its own.

The second sub-assembly comprises a second inner clamp 110b, a second outer clamp 130b and a second nut 150a. The second inner clamp 110b is comprised of a lower portion 111b and an upper portion 118b. The lower portion 111b comprises a pair of tongs or fingers 112, 114 that are capable of gripping a second rod member 10 therebetween. In some embodiments, the inner clamp 110b is capable of being top-loaded onto a second rod member. The second rod member can also be viewed as being bottom-loaded into the inner clamp 110b. The upper portion 118b of the inner clamp 110b comprises a threaded cylindrical portion that is capable of engaging inner threads of the second nut 150b. Rotation of the second nut 150b causes the fingers 112, 114 of the inner clamp 110b to close on the second rod member, as will be discussed in further detail below. In some embodiments, an upper section of the threaded upper portion 118b is distorted or peened over, thereby preventing a nut 150b from loosening from the threaded upper portion 118b.

The second outer clamp 130b comprises a first clamping portion 132 and a second clamping portion 134. As shown in FIG. 3, the first clamping portion 132 comprises an inner wall designed to contact an outer surface of the finger 112 of the second inner clamp 110b, while the second clamping portion 134 comprises an inner wall designed to contact an outer surface of the finger 114 of the second inner clamp 110b. Rotation of the second nut 150b causes the second inner clamp 110b to be drawn upward. As the second inner clamp 110b is drawn upward, the inner walls of the second outer clamp 130b engage the outer walls of the second inner clamp 110b, thereby compressing the second inner clamp 110b onto a second rod member that is received therein.

The second nut 150b comprises an inner threaded section that is configured to engage the outer threads of the upper portion 118b of the second inner clamp 110b. In some embodiments, rotation of the second nut 150b in a first direction draws the second inner clamp 110a upward toward the inner walls of the second outer clamp 130b, thereby causing the fingers 112, 114 of the second inner clamp 110b to be compressed onto a first rod member 10. Rotation of the first nut 150b in a second opposite direction translates the second inner clamp 110b downward and away from the inner walls of the second outer clamp 130b, thereby causing the fingers 112, 114 of the second inner clamp 110b to release from the second rod member 10 if desired. In some embodiments, an upper surface of the second nut 150b is substantially smooth, while a lower surface of the second nut 150b comprises a more textured surface. In some embodiments, the lower surface of the second nut 150b comprises a star grind. Advantageously, when the second nut 150b is fully tightened, the star grind provides resistance against an upper surface of the cross rod 170, thereby reducing the likelihood of the second nut 150b unintentionally rotating backwards and loosening on its own.

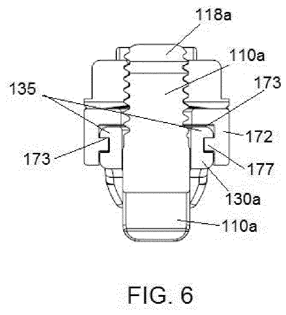

A cross rod 170 extends and is operably connected to the first sub-assembly and the second sub-assembly. The cross rod 170 comprises a left portion 172 comprising a first slot 178a through which the first sub-assembly can extend and a right portion 174 comprising a second slot 178b through which the second sub-assembly can extend. Each of the sub-assemblies is connected to the cross rod 170 in the same manner. FIG. 6 illustrates how one of the sub-assemblies is connected to the cross rod 170. The other sub-assembly is connected in the same manner. In particular, the first outer clamp 130a comprises one or more rails 135 that are received within one or more recesses 173 of the cross rod 170. By providing such a rail feature, the first sub-assembly is advantageously capable of translating and sliding relative to the cross rod 170 within the first slot 178a, while the second sub-assembly is advantageously capable of translating and sliding relative to the cross rod 170 within the second slot 178b. This translational movement of both of the sub-assemblies within the slots 178a, 178b allows the transconnector to accommodate rod members of varying distances. Note that the length of the cross rod itself does not change length. In some embodiments, each of the sub-assemblies is capable of translating up to 3 mm, while in other embodiments, the sub-assemblies are capable of translating up to 5 mm or 7 mm or more. In addition, in some embodiments, the sub-assemblies are advantageously capable of slight angulation within the slots 178a, 178b, thereby the cross rod 170 to attached to non-parallel rod members.

As shown in FIG. 2, the cross rod 170 includes a raised, vertically arched portion 176 that extends between the left portion 172 and the right portion 174. The arched portion 176 advantageously accommodates any portion of the vertebrae that may protrude outwardly, such as the spinous process or portions thereof. For example, as shown in FIG. 1, the transconnector extends over spinous processes which have been removed. By providing an arched portion 176, the cross rod 170 is capable of accommodating any remaining portions of a spinous process that remain on the vertebrae. Advantageously, the arched portion 176 does not have any type of nut or set screw extending through it. When a nut or set screw is provided medial to rod members 10 (as opposed to on top of them as in the present application), there is a risk that a doctor rotating the nut or set screw could jab into an exposed spinal cord via a hand or instrument. The transconnector 100 of the present application reduces this risk by providing nuts 150a, 150b that are directly above rod members 10, and not medial to their respective rod members 10.

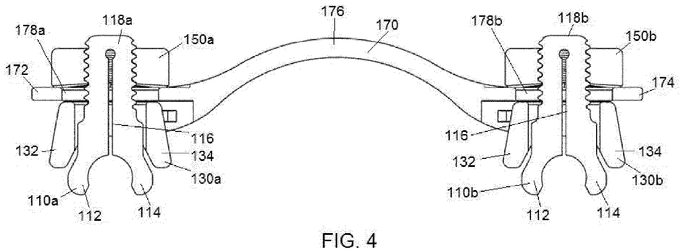

FIG. 3 shows a side view of the transverse connector of FIG. 2. From this views, one can see the profiles of the inner clamps 110a, 110b. First inner clamp 110a comprises a threaded upper portion 118a and a lower portion including a pair of fingers 112, 114 for gripping a first rod member therein. The pair of fingers 112, 114 are separated via a slit 116. The slit advantageously allows the fingers 112, 114 to provisionally grip and clamp onto a first rod member even before the first nut 150a is tightened. Likewise, second inner clamp 110b comprises a threaded upper portion 118b and a lower portion including a pair of fingers 112, 114 for gripping a second rod member therein. The pair of fingers 112, 114 are separated via a slit 116. The slit advantageously allows the fingers 112, 114 to slightly splay, thereby provisionally gripping and clamping onto a second rod member even before the second nut 150b is tightened. In some embodiments, a slit 116 can extend into the upper threaded portion 118a, 118b of the inner clamp 110a, 110b (as shown in FIG. 4), thereby advantageously allowing the inner clamp 110a, 110b to have an enhanced splaying feature when gripping onto a rod member.

FIG. 4 shows a side cross-sectional view of the transverse connector of FIG. 2. In FIG. 4, the first sub-assembly including the inner clamp 110a, outer clamp 130a and nut 150a is in an open configuration, while the second assembly including the inner clamp 110b, outer clamp 130b and nut 150b is in a closed configuration. In the open configuration, the fingers 112, 114 of the inner clamp 110a are uncompressed by the inner walls of the outer clamp 130a, such that the inner clamp 110a is capable of receiving a rod member therein. In the closed configuration, the nut 150b has been rotated, thereby causing the inner clamp 110b to be drawn upwardly into the outer clamp 130b. As the inner clamp 110b is drawn upwardly, the fingers 112, 114 of the inner clamp 110b become compressed by the inner walls of the outer clamp 130b, such that any rod member received in the inner clamp 110b would be tightly clamped.

From the cross-sectional view of FIG. 4, one can also see the slots 178a, 178b through which the first sub-assembly and the second sub-assembly are capable of translating. Advantageously, the slots 178a, 178b allow the transverse connector to attach to rod members 10 of varying distance.

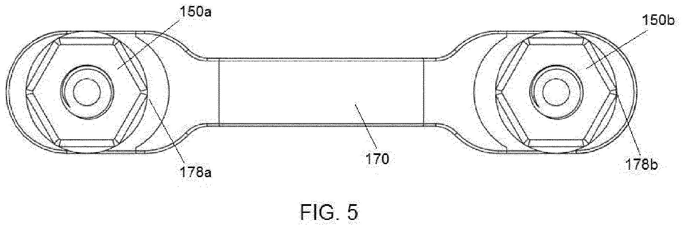

FIG. 5 shows a top view of the transverse connector of FIG. 2. From this view, one can see a top view of the slots 178a, 178b through which the first sub-assembly and the second sub-assembly are capable of translating.

FIG. 6 shows a front cross-sectional view of the transverse connector of FIG. 2. From this view, one can see how the rails 135 of the outer clamp 130a engage the slots or recesses 173 formed in the inner walls of the cross rod 170. In some embodiments, one or more rails 135 of the outer clamp 130a are capable of sliding one or more corresponding rail portions 177 that extend radially from an inner wall of the cross rod 170.

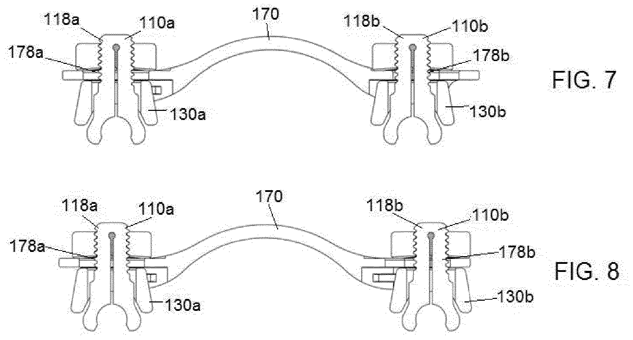

FIG. 7 shows a side cross-sectional view of the transverse connector of FIG. 2, whereby the sub-assemblies are separated a first distance. FIG. 8 shows a side cross-sectional view of the transverse connector of FIG. 2, whereby the sub-assemblies are separated a second distance different from the first distance in FIG. 7. As shown in the figures, the slots 178a, 178b enable the sub-assemblies including the inner clamp, outer clamp and nut to translate, thereby accommodating rod members 10 that are of varying distance relative to one another.

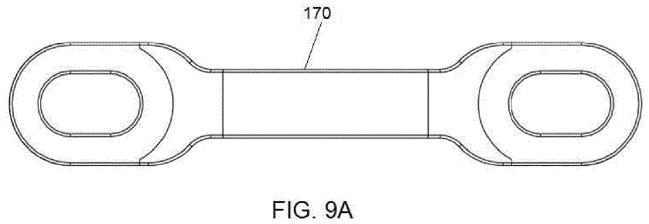

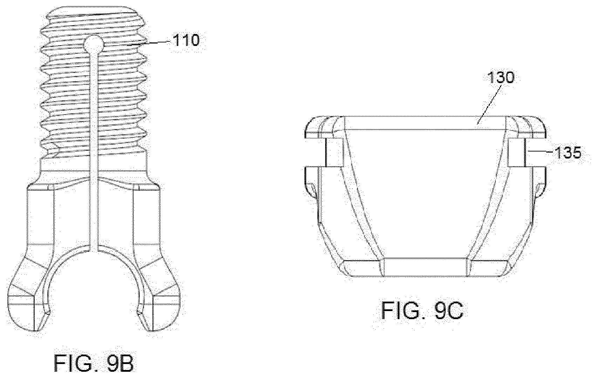

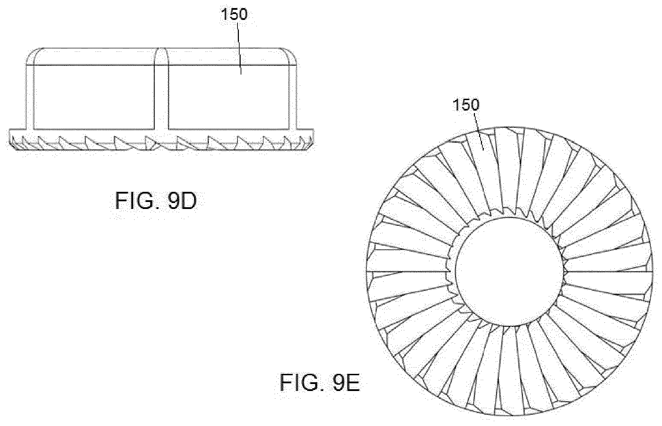

FIGS. 9A-9E show different views of individual components of the transverse connector of FIG. 2. FIG. 9A shows a top view of a cross rod 170 including elongated slots 178a, 178b. FIG. 9B shows a front view of an inner clamp 110. FIG. 9C shows a front view of an outer clamp 130 including rails 135 for sliding relative to the cross rod 170. FIG. 9D shows a front view of the nut 150, while FIG. 9E shows a bottom view of the nut 150 including the star grind, in accordance with some embodiments.

A method of using the improved transconnector 100, which is not part of the invention, is now described. A surgeon can implant a first rod member 10 into a pair of tulip heads of screws and a second rod member 10 into a pair of tulip heads of screws (as shown in FIG. 1). The surgeon can then deliver the transconnector 100 over each of the first and second rod members 10. The transconnector 100 comprises a pair of subassemblies (an inner clamp 110, an outer clamp 130 and a nut 150) that are received in respective slots 178 formed in a cross rod 170 of the transconnector 100. The subassemblies are capable of separating varying distances from one another, thereby allowing the transconnector 100 to accommodate rod members 10 of varying distance relative to one another. Furthermore, the subassemblies are capable of angulating relative to the cross rod 170, thereby allowing the transconnector 100 to accommodate rod members 10 of different angulations relative to one another. Once the inner clamps 110 are provisionally clamped onto their respective rods, the nuts 150 can be rotated, thereby further tightening the inner clamps 110 on the rods.

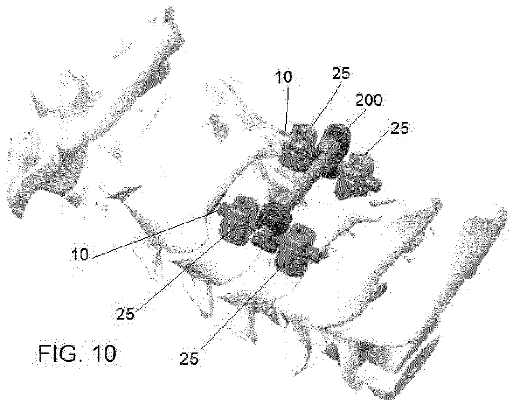

FIG. 10 shows an embodiment of a stabilization system using an alternative transverse connector in accordance with some embodiments. The stabilization system comprises a first rod member 10 received in first and second screws (e.g., pedicle screws) 25, and a second rod member 10 received in third and fourth screws (e.g., pedicle screws) 25. The first rod member 10 is positioned on one side of a spine, while the second rod member 10 is positioned on the other side of the spine. An improved transverse connector 200 extends between the first rod member 10 and the second rod member 10. In some embodiments, the first rod member 10 and the second rod member 10 can be side-loaded into mouths of the transverse connector 200. The transverse connector 200 is advantageously capable of accommodating rod members having varying lengths of separation, as well as rod members that are non-parallel to one another.

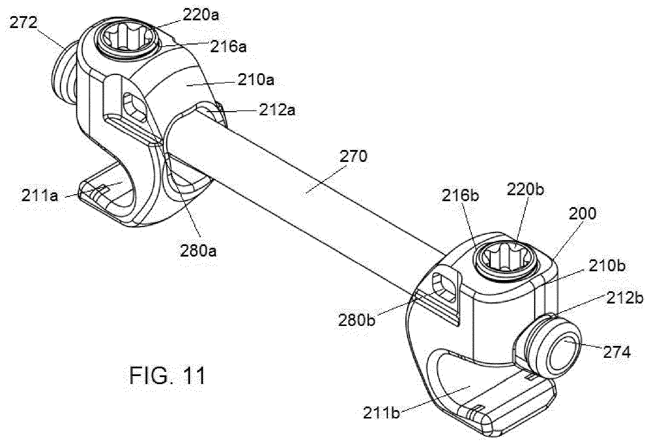

FIG. 11 shows a perspective view of a transverse connector in accordance with some embodiments. On one end, the transverse connector 200 comprises a first sub-assembly comprising a first clamp body 210a for receiving a first rod member and a first set screw 220a extending through an opening 216a formed through an upper surface of the body. On the other end, the transverse connector 200 comprises a second sub-assembly comprising a second clamp body 210b for receiving a second rod member and a second set screw 220b extending through an opening 216b formed through an upper surface of the body. The first sub-assembly and the second sub-assembly are connected to one another via a cross rod 270 that extends therebetween.

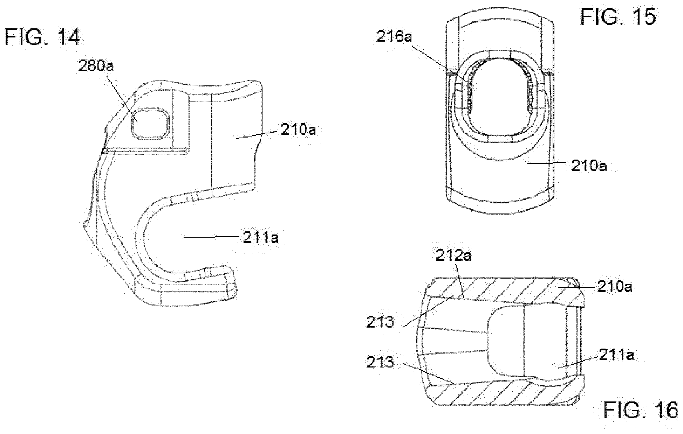

The first sub-assembly comprises a first clamp body 210a and a set screw 220a extending through an opening 216a formed in the body. The first clamp body 210a comprises a side slot or mouth 211a for receiving a first rod member 10 therein. Advantageously, the first rod member 10 is capable of side-loading into the first clamp body 210a. The first clamp body 210a further comprises a side opening 212a that extends through opposed sidewalls of the first clamp body 210a. The side opening 212a is capable of receiving the cross rod 270 therethrough. Advantageously, opposed inner walls 213 that form the side opening 212a (shown in FIG. 16) can be non-parallel or at an angle relative to one another. The angulation of these inner walls advantageously allows the first clamp body 210a to angulate relative to the cross rod 270 and thereby accept a first rod member 10 that may be non-parallel to a second rod member 10. The first clamp body 210a is advantageously capable of sliding relative to the cross rod 270, thereby accommodating first and second rod members 10 of different distance relative to one another. The first clamp body 210a further comprises a top opening 216a that extends through an upper wall of the first clamp body 210a. The top opening 216a is capable of receiving a set screw 220a therein. The set screw 220a can be downwardly threaded and tightened, thereby locking into place the relative position and orientation of the first clamp body 210a along the cross rod 270.

As shown in FIG. 11, the first clamp body 210a can include one or more tool gripping surfaces 280a. In some embodiments, the first clamp body 210a includes a pair of tool gripping surfaces 280a formed on each of the sidewalls of the first clamp body 210a. In some embodiments, the tool gripping surfaces 280a can comprise a recessed portion, wherein within the recessed portion a further indentation is formed. By providing such gripping surfaces 280a, this advantageously allows an instrument to securely hold onto the first clamp body 210a during implantation.

The second sub-assembly comprises a second clamp body 210b and a set screw 220b extending through an opening 216a formed in the body. The second clamp body 210b comprises a side slot or mouth 211a for receiving a second rod member 10 therein. Advantageously, the second rod member 10 is capable of side-loading into the second clamp body 210b. The second clamp body 210b further comprises a side opening 212b that extends through opposed sidewalls of the second clamp body 210b. The side opening 212b is capable of receiving the cross rod 270 therethrough. Advantageously, opposed inner walls 213 that form the side opening 212b can be non-parallel or at an angle relative to one another. The angulation of these inner walls advantageously allows the second clamp body 210b to angulate relative to the cross rod 270 and thereby accept a second rod member 10 that may be non-parallel to a first rod member 10. The second clamp body 210b is advantageously capable of sliding relative to the cross rod 270, thereby accommodating first and second rod members 10 of different distance relative to one another. The second clamp body 210b further comprises a top opening 216b that extends through an upper wall of the second clamp body 210b. The top opening 216b is capable of receiving a set screw 220b therein. The set screw 220b can be downwardly threaded and tightened, thereby locking into place the relative position and orientation of the second clamp body 210b along the cross rod 270.

As shown in FIG. 11, the second clamp body 210b can include one or more tool gripping surfaces 280b. In some embodiments, the second clamp body 210b includes a pair of tool gripping surfaces 280b formed on each of the sidewalls of the second clamp body 210b. In some embodiments, the tool gripping surfaces 280b can comprise a recessed portion, wherein within the recessed portion a further indentation is formed. By providing such gripping surfaces 280b, this advantageously allows an instrument to securely hold onto the second clamp body 210b during implantation.

The cross rod 270 extends between the first clamp body 210a and the second clamp body 210b. The cross rod 270 comprises a cylindrical body having enlarged ends 272, 274. The enlarged ends 272, 274, which have a greater diameter than the intermediate cross rod 270 body, reduce the likelihood of the first clamp body 210a and the second clamp body 210b being dismantled from the transconnector 200.

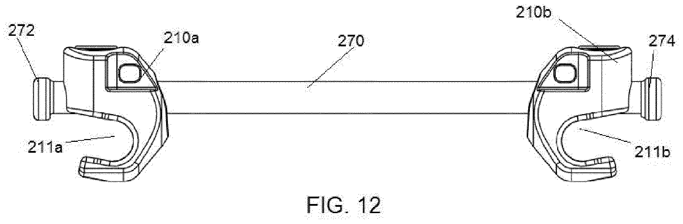

FIG. 12 shows a side view of the transverse connector of FIG. 11. From this view, one can see the distinct shapes of the side mouths 211a, 211b of the first and second clamp bodies 210a, 210b. Each of the mouths 211a, 211b comprises an angled upper surface that tapers downwardly to a convex region for receiving a rod member therein. A downward surface also tapers downwardly to the convex region, thereby advantageously creating a distinct recess for receiving a rod member securely therein.

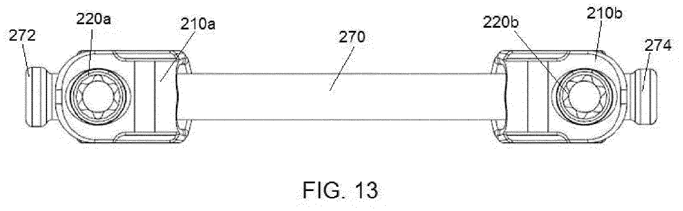

FIG. 13 shows a top view of the transverse connector of FIG. 11. From this view, one can see the set screws 220a, 220b that extend downwardly into the first and second clamp bodies 210a, 210b. In some embodiments, the set screws 220a, 220b are threaded such that they can be downwardly threaded to tighten onto the cross rod 270. In other embodiments, the set screws 220a, 220b are non-threaded. The non-threaded set screws 220a, 220b can include, for example, protrusions that can be received into slots, whereby rotation of the set screws 220a, 220b locks the clamp bodies 210a, 210b relative to the cross rod 270.

FIG. 14 shows a side cross-sectional view of a clamp body of the transverse connector of FIG. 11. From this view, one can see the tool gripping surface 280a, whereby the tool gripping surface is formed of a recess and a further indentation within the recess.

FIG. 15 shows a front cross-sectional view of a clamp body of the transverse connector of FIG. 11. From this view, one can see the opening 216a through which a set screw is received therein.

FIG. 16 shows top cross-sectional view of a clamp body of the transverse connector of FIG. 11. From this view, one can see the angled walls 213 that form the side opening 212a. In some embodiments, the angled walls 213 are non-parallel to one another, thereby allowing the clamp body 210a to angulate relative to the cross rod 270 and accept a rod member 10 of various angles.

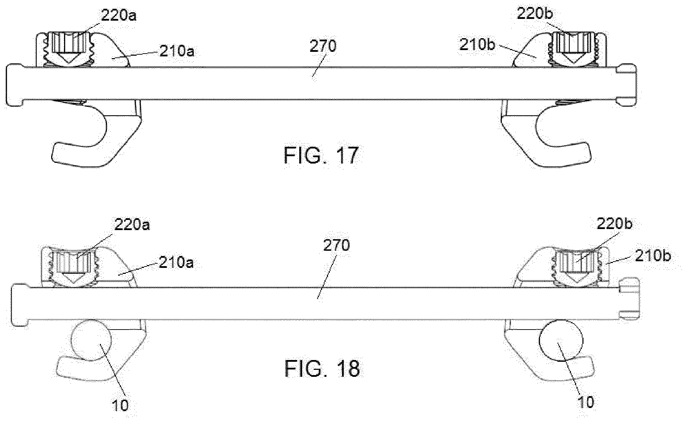

FIG. 17 shows a side cross-sectional view of the transverse connector of FIG. 11 without rod members received therein, while FIG. 18 shows a side cross-sectional view of the transverse connector of FIG. 10 with rod members received therein. As shown in FIG. 18, the cross rod 270 is in contact with each of first and second rod members 10. On the left side of the cross rod 270, rotation of the set screw 220a causes downward compression on the cross rod 270, which in turn compresses the first rod member 10. On the right side of the cross rod 270, rotation of the set screw 220b causes downward compression on the cross rod 270, which in turn compresses the second rod member 10.

A method of using the improved transconnector 200, which is not part of the invention, is now described. A surgeon can implant a first rod member 10 into a pair of tulip heads of screws and a second rod member 10 into a pair of tulip heads of screws (as shown in FIG. 10). The surgeon can then deliver the transconnector 200 to the mplant site, wherein each of the first and second rod members 10 can be side-loaded into clamps of the transconnector. The transconnector 200 comprises a pair of subassemblies (a clamp body 210 including a side mouth and a set screw 220). A cross rod 270 can extend between the pair of subassemblies. The subassemblies are capable of separating varying distances from one another, thereby allowing the transconnector 200 to accommodate rod members 10 of varying distance relative to one another. Furthermore, the subassemblies are capable of angulating relative to the cross rod 270, thereby allowing the transconnector 200 to accommodate rod members 10 of different angulations relative to one another. Once the clamp bodies 210 are provisionally clamped onto their respective rods, the set screws 220 can be rotated. Rotation of the set screws 220 applies downward pressure on the cross rod 270, which applies downward pressure on the rod members 10, thereby fixing the orientation of the transconnector 200 relative to the rod members 10.

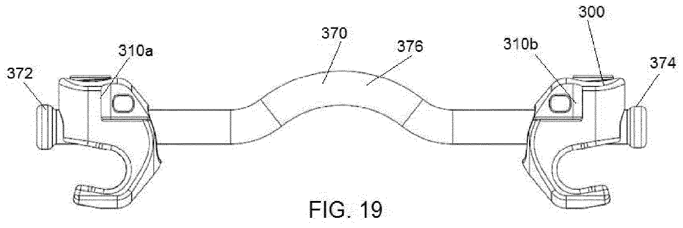

FIG. 19 shows an alternative transconnector in accordance with some embodiments. Like the transconnector 200, the transconnector 300 comprises a first clamp body 310a, a second clamp body 310b and a cross rod 370. However, in the present embodiment, the cross rod 370 has an arched, vertically raised intermediate portion 376. The advantage of providing such an arched portion 376 is that it accommodates any spinous process portions that may remain.

Each of the transconnectors described above can be used with various types of stabilization systems, including rods, screws (e.g., pedicle screws), and plates. In addition, the transconnectors can be used with various implants, including implants (e.g., fusion cages and spacers) and prosthetics.

## Claims

1. A surgical system comprising:
a first rod member (10);
a second rod member (10); and
a transverse connector (100) operably attached to the first rod member (10) and the second rod member (10), wherein the transverse connector (100) comprises a cross rod (170), a first sub-assembly (110a, 130a, 150a) for gripping onto the first rod member 10 and a second sub-assembly (110b, 130b, 150b) for gripping onto the second rod member (10), wherein the first rod member(10) is bottom loaded onto the first sub-assembly (110a, 130a, 150a) and the second rod member is bottom loaded onto the second sub-assembly (110b, 130b, 150b),
wherein the first sub-assembly (110a, 130a, 150a) comprises a first inner clamp (110a) and a first outer clamp (130a) and a first nut (150a) that extends around the upper threaded portion (118a) of the first inner clamp (110a), wherein a lower surface of the first nut (150a) includes a star grind;
wherein the first inner clamp (110a) comprises an upper threaded portion (118a) and a lower portion 111b comprising a first pair of fingers (112,114),
wherein a slit (116) separates the first pair of fingers (112, 114) and extends into the upper threaded portion (118a) of the first inner clamp (110a) and wherein the star grind is so configured that when the first nut (150a) is fully tightened, the star grind provides resistance against an upper surface of the cross rod (170).

2. The surgical system of claim 1, wherein the second sub-assembly (110b, 130b, 150b) comprises a second inner clamp (110b) and a second outer clamp (130b).

3. The surgical system of claim 2, wherein the second inner clamp (110b) comprises a second upper threaded portion (118b) and a second lower portion (111b) comprising a second pair of fingers (112, 114).

4. The surgical system of claim 2, wherein the second inner clamp (110b) comprises a slit (116) that separates the second pair of fingers (112, 114).

5. The surgical system of claim 1, wherein the cross rod (170) comprises an arched portion (176) that extends between the first sub-assembly (110)(110a, 130a, 150a) and the second sub-assembly (110b, 130b, 150b).

6. The surgical system of claim 5, wherein the first sub-assembly (110a, 130a, 150a) is received in a first slot ((178a)) formed in the cross rod (170) and the second sub-assembly (110b, 130b, 150b) is received in a second slot ((178b)) formed in the cross rod (170).

7. The surgical system of claim 6, wherein the first sub-assembly (110a, 130a, 150a) is capable of translation in the first slot (178a) and the second sub-assembly (110b, 130b, 150b) is capable of translation in the second slot (178b).

8. The surgical system of claim 7, wherein the first sub-assembly (110a, 130a, 150a) is capable of translating up to 3 mm within the first slot (178a).

9. The surgical system of claim 8, wherein the second sub-assembly (110b, 130b, 150b) is capable of translating up to 3 mm within the second slot (178b).

10. The surgical system of claim 9, wherein the first sub-assembly (110a, 130a, 150a) comprises a first rail (135) that extends through the first slot (178a).

11. The surgical system of claim 10, wherein the transverse connector (100) comprises a first recess 173 to receive the first rail (135).

12. The surgical system of claim 11, wherein the second sub-assembly (110b, 130b, 150b) comprises a second rail (135) that extends through the second slot (178b).

13. The surgical system of claim 12, wherein the transverse connector (100) comprises a second recess to receive the second rail (135).

14. The surgical system of claim 1 further comprising a first pedicle screw (25) configured to secure the first rod member (10) to a vertebral body and a second pedicle screw (25) configured to secure the second rod member (10) to a vertebral body.

## Patentansprüche

1. Chirurgisches System, umfassend:
ein erstes Stabelement (10);
ein zweites Stabelement (10); und
einen Querverbinder (100), der mit dem ersten Stabelement (10) und dem zweiten Stabelement (10) wirkverbunden ist, wobei der Querverbinder (100) eine Querstrebe (170), eine erste Unterbaugruppe (110a, 130a, 150a) zum Ergreifen des ersten Stabelements (10) und eine zweite Unterbaugruppe (110b, 130b, 150b) zum Ergreifen des zweiten Stabelements (10) umfasst, wobei das erste Stabelement (10) von unten in die erste Unterbaugruppe (110a, 130a, 150a) zugeführt wird und das zweite Stabelement von unten in die zweite Unterbaugruppe (110b, 130b, 150b) zugeführt wird,
wobei die erste Unterbaugruppe (110a, 130a, 150a) eine erste innere Klemme (110a) und eine erste äußere Klemme (130a) und eine erste Mutter (150a) umfasst, die sich um den oberen Gewindeabschnitt (118a) der ersten inneren Klemme (110a) erstreckt, wobei eine Unterseite der ersten Mutter (150a) einen Sternschliff aufweist;
wobei die erste innere Klemme (110a), einen oberen Gewindeabschnitt (118a) und einen unteren Abschnitt (111b) mit einem ersten Paar Finger (112, 114) umfasst,
wobei ein Schlitz (116) das erste Paar Finger (112, 114) trennt und sich in den oberen Gewindeabschnitt (118a) der ersten inneren Klemme (110a) erstreckt, und wobei der Sternschliff so konfiguriert ist, dass, wenn die erste Mutter (150a) vollständig angezogen ist, der Sternschliff einen Widerstand gegen eine Oberseite der Querstrebe (170) bereitstellt.

2. Chirurgischen System nach Anspruch 1, wobei die zweite Unterbaugruppe (110b, 130b, 150b) eine zweite innere Klemme (110b) und eine zweite äußere Klemme (130b) umfasst.

3. Chirurgisches System nach Anspruch 2, wobei die zweite innere Klemme (110b) einen zweiten oberen Gewindeabschnitt (118b) und einen zweiten unteren Abschnitt (111b) mit einem zweiten Paar Finger (112, 114) umfasst.

4. Chirurgische System nach Anspruch 2, wobei die zweite innere Klemme (110b) einen Schlitz (116) aufweist, der das zweite Paar Finger (112, 114) trennt.

5. Chirurgisches System nach Anspruch 1, wobei die Querstrebe (170) einen gebogenen Abschnitt (176) umfasst, der sich zwischen der ersten Unterbaugruppe (110) (110a, 130a, 150a) und der zweiten Unterbaugruppe (110b, 130b, 150b) erstreckt.

6. Chirurgisches System nach Anspruch 5, wobei die erste Unterbaugruppe (110a, 130a, 150a) in einem ersten Schlitz (178a) aufgenommen ist, der in der Querstrebe (170) ausgebildet ist, und die zweite Unterbaugruppe (110b, 130b, 150b) in einem zweiten Schlitz (178b) aufgenommen ist, der in der Querstrebe (170) ausgebildet ist.

7. Chirurgisches System nach Anspruch 6, wobei die erste Unterbaugruppe (110a, 130a, 150a) im ersten Schlitz (178a) verschiebbar ist und die zweite Unterbaugruppe (110b, 130b, 150b) im zweiten Schlitz (178b) verschiebbar ist.

8. Chirurgisches System nach Anspruch 7, wobei die erste Unterbaugruppe (110a, 130a, 150a) bis zu 3 mm innerhalb des ersten Schlitzes (178a) verschiebbar ist.

9. Chirurgisches System nach Anspruch 8, wobei die zweite Unterbaugruppe (110b, 130b, 150b) bis zu 3 mm innerhalb des zweiten Schlitzes (178b) verschiebbar ist.

10. Chirurgisches System nach Anspruch 9, wobei die erste Unterbaugruppe (110a, 130a, 150a) eine erste Schiene (135) umfasst, die sich durch den ersten Schlitz (178a) erstreckt.

11. Chirurgisches System nach Anspruch 10, wobei der Querverbinder (100) eine erste Ausnehmung 173 zum Aufnehmen der ersten Schiene (135) umfasst.

12. Chirurgisches System nach Anspruch 11, wobei die zweite Unterbaugruppe (110b, 130b, 150b) eine zweite Schiene (135) umfasst, die sich durch den zweiten Schlitz (178b) erstreckt.

13. Chirurgisches System nach Anspruch 12, wobei der Querverbinder (100) eine zweite Ausnehmung zum Aufnehmen der zweiten Schiene (135) umfasst.

14. Chirurgisches System nach Anspruch 1, das ferner eine erste Pedikelschraube (25), die zum Befestigen des ersten Stabelements (10) an einem Wirbelkörper konfiguriert ist, und eine zweite Pedikelschraube (25) umfasst, die zum Befestigen des zweiten Stabelements (10) an einem Wirbelkörper konfiguriert ist.

## Revendications

1. Système chirurgical comprenant :
un premier élément de tige (10) ;
un second élément de tige (10) ; et
un raccord transversal (100) fixé fonctionnellement au premier élément de tige (10) et au second élément de tige (10), dans lequel le raccord transversal (100) comprend une tige transversale (170), un premier sous-ensemble (110a, 130a, 150a) destiné à s'accrocher sur le premier élément de tige (10) et un second sous-ensemble (110b, 130b, 150b) destiné à s'accrocher sur le second élément de tige (10), dans lequel le premier élément de tige (10) est chargé par le fond sur le premier sous-ensemble (110a, 130a, 150a) et le second élément de tige est chargé par le fond sur le second sous-ensemble (110b, 130b, 150b),
dans lequel le premier sous-ensemble (110a, 130a, 150a) comprend une première attache interne (110a) et une première attache externe (130a) et un premier écrou (150a) qui s'étend autour de la portion filetée supérieure (118a) de la première attache interne (110a), dans lequel une surface inférieure du premier écrou (150a) comporte un fraisage en étoile ;
dans lequel la première attache interne (110a) comprend une portion filetée supérieure (118a) et une portion inférieure (111b) comprenant une première paire de doigts (112, 114),
dans lequel une entaille (116) sépare la première paire de doigts (112, 114) et s'étend dans la portion filetée supérieure (118a) de la première attache interne (110a) et dans lequel le fraisage en étoile est configuré de sorte que lorsque le premier écrou (150a) est complètement serré, le fraisage en étoile fournisse une résistance contre une surface supérieure de la tige transversale (170).

2. Système chirurgical selon la revendication 1, dans lequel le second sous-ensemble (110b, 130b, 150b) comprend une seconde attache interne (110b) et une seconde attache externe (130b).

3. Système chirurgical selon la revendication 2, dans lequel la seconde attache interne (110b) comprend une seconde portion filetée supérieure (118b) et une seconde portion inférieure (111b) comprenant une seconde paire de doigts (112, 114).

4. Système chirurgical selon la revendication 2, dans lequel la seconde attache interne (110b) comprend une entaille (116) qui sépare la seconde paire de doigts (112, 114).

5. Système chirurgical selon la revendication 1, dans lequel la tige transversale (170) comprend une portion en arche (176) qui s'étend entre le premier sous-ensemble (110) (110a, 130a, 150a) et le second sous-ensemble (110b, 130b, 150b).

6. Système chirurgical selon la revendication 5, dans lequel le premier sous-ensemble (110a, 130a, 150a) est reçu dans une première fente (178a) formée dans la tige transversale (170) et le second sous-ensemble (110b, 130b, 150b) est reçu dans une seconde fente (178b) formée dans la tige transversale (170).

7. Système chirurgical selon la revendication 6, dans lequel le premier sous-ensemble (110a, 130a, 150a) est capable d'une translation dans la première fente (178a) et le second sous-ensemble (110b, 130b, 150b) est capable d'une translation dans la seconde fente (178b).

8. Système chirurgical selon la revendication 7, dans lequel le premier sous-ensemble (110a, 130a, 150a) est capable d'une translation jusqu'à 3 mm à l'intérieur de la première fente (178a).

9. Système chirurgical selon la revendication 8, dans lequel le second sous-ensemble (110b, 130b, 150b) est capable d'une translation jusqu'à 3 mm à l'intérieur de la seconde fente (178b).

10. Système chirurgical selon la revendication 9, dans lequel le premier sous-ensemble (110a, 130a, 150a) comprend un premier rail (135) qui s'étend à travers la première fente (178a).

11. Système chirurgical selon la revendication 10, dans lequel le raccord transversal (100) comprend un premier évidement (173) pour recevoir le premier rail (135).

12. Système chirurgical selon la revendication 11, dans lequel le second sous-ensemble (110b, 130b, 150b) comprend un second rail (135) qui s'étend à travers la seconde fente (178b).

13. Système chirurgical selon la revendication 12, dans lequel le raccord transversal (100) comprend un second évidement pour recevoir le second rail (135).

14. Système chirurgical selon la revendication 1, comprenant en outre une première vis pédiculaire (25) configurée pour fixer le premier élément de tige (10) à un corps vertébral et une seconde vis pédiculaire (25) configurée pour fixer le second élément de tige (10) à un corps vertébral.
